# EUROPEAN PATENT SPECIFICATION

(11) **EP 2 338 077 B1**
(45) Date of publication and mention of the grant of the patent: **02.08.2017**
(21) Application number: 09752719.6
(22) Date of filing: 14.09.2009
(51) Int. Cl.: G02B 21/08, A61B 5/103, A61B 5/00, A61B 90/30

(54) **ILLUMINATION MODULE FOR OPTICAL SYSTEMS WITH ANGULAR LIGHT SHUTTER**
BELEUCHTUNGSMODUL FÜR OPTISCHE SYSTEME MIT WINKELLICHTBLENDE
MODULE D'ILLUMINATION POUR DES SYSTÈMES OPTIQUES DOTÉS D'UN OBTURATEUR DE LUMIÈRE ANGULAIRE

(30) Priority: 15.09.2008 IT TP20080009
(43) Date of publication of application: 29.06.2011
(73) Proprietor: Adamo, Alessio, Trapani (IT)
(72) Inventor: Adamo, Alessio, Trapani (IT)
(86) International application number: PCT/EP2009/006637
(87) International publication number: WO 2010/028854

(56) References cited:
- EP-A1- 0 541 808
- GB-A- 1 321 783
- GB-A- 1 407 870
- GB-A- 2 364 376
- GB-A- 2 364 567
- US-A- 3 770 342
- US-A- 3 944 341
- US-A- 5 971 919

## Description

This MODULE finds a practical utilization in medical diagnostics, particularly in dermatology, in the examination of skin lesions. Binocular optical systems (stereomicroscopes) are really appreciated and used in non-invasive diagnostics in gynaecology thanks to the excellent quality of their image and are constantly updated. In dermatologic diagnostics they have always been poorly used because of their poor ease of use and poor efficiency. The main purpose of this module is to make binocular optical systems suitable for the dermatological field, thus filling all those technological gaps that made them unsuitable for this purpose, and to introduce new diagnostic horizons.

All the other optical systems for dermoscopy, methodology which deals with skin lesions diagnosis, are divided into two groups: monocular and binocular systems. Several typologies of products have been developed in the monocular version. The monocular version is characterized by building simplicity and reduced dimensions, which permit an easy and quick use. In the last years many digital systems have been developed that have the advantage of being very compact but that also have important limitations determined by the image flattening (two dimensions), typical of monocular systems, with a total absence of the depth of field, and by a little penetrating lightning (led diodes) in addition to the limited definition of the digital image obtained by the systems used at present. The binocular version is basically composed by regular stereomicroscopes developed for microsurgery and lent to dermatologic diagnostics. The binocular systems used at present are the only systems that permit the tridimensional visualization of the image, a feature that is very important to evaluate precisely how spread the lesion is in depth (third dimension) and therefore beyond the epidermis. These systems, lacking some features that are fundamental for epiluminescence dermatoscopy and that are widely described in literature, do not permit to easily and safely perform a dermoscopic examination and are therefore rarely used. The main inconveniences of this present technique are:
1. The difficult positioning of the stereomicroscope, due to the necessity of keeping a well-defined work distance (or focal length), since these systems lack a bracket that may permit the correct positioning, necessary to keep the exact focusing of the image constant in time;
2.the necessity of applying oil or gel on a glass to eliminate the surface refraction of light;
3.the lightning of the area to be examined is widespread and perpendicular, without a specific angle of incidence with the surface able to penetrate properly the horny layer, as provided for by epiluminescence dermatoscopy;
4.the remarkable difficulty in capturing images (cameras, digital modules) with a perfect focusing of the area to be examined (due to the small movements of the operator and of the patient), feature that is important for the follow-up;
5.the loss of tridimensionality, due to the use of digital systems, indeed very useful for a correct optical evaluation of the depth (third dimension) of the skin lesion.
An illumination system for a microscope wherein illumination is provided via optical fibres is disclosed in the UK patent application published on 24 September 1975 with publication number GB 1407870.

### Brief description of the drawings:

Tavola "A" (i.e. Figure A) - hereafter referred to as Tab. "A" - depicts three views of a longitudinal section of the illumination module of the current invention and both a longitudinal section and a cross-section of the concentrator in which the optical fibres of the module are arranged in a ring of optical fibres. This module, assembled with a binocular optical system and properly connected to a light source, permits to obtain the following advantages:
1.immediate positioning of the device on the area to be examined thanks to the specific conformation of the MODULE and to the presence of a non-reflective glass (7/Tab."A") fixed to the end of the same module;
2.correct angle of incidence of light (about 30°) determined by the positioning of the fibre optics in the concentrator (5/Tab."A"), inside the module, near the contact area;
3.proper lightning of the area to be examined thanks to the ring-shaped layout of the optical fibre bundle that conveys all the available light power in the interested area;
4.shuttering of light in a well-defined arc portion of the fibre optics ring (9/Tab."A") ;
5.immediate and constant focusing of the area to be examined, which makes the examination quick and safe and which permits a fast and perfect capturing with photo and digital systems (videodermoscopy)
6.excellent tridimensional visualization of the lesion (stereoscopy, angle of incidence of light, proper lightning), which permits a correct evaluation of the depth of the skin lesion, thus optimizing the diagnosis.

The CONTACT MODULE (1/Tab."A") has a conical shape on the outside and can be built with different dimensions, according to the work distance of the optical system in use and to the contact surface (poor accessible areas). The optical fibre bundle (6/Tab."A") is equally spread, at one of the ends, along a circumference by a concentrator (5/Tab."A"), which determines the angle of incidence of the light beam with the contact surface. The position of the concentrator can be adjusted to modify the surface of incidence of the light beam and to obtain a perfect matching with the optical system in use since, modifying the
magnification, we can obtain an inversely proportional variation of the field of view and therefore a variation of the surface to be lightened. The other end of the optical fibre bundle is inserted inside a cylindrical connector (2/Tab."A"), properly pierced, according to an exact layout, in a way to create a spiral with the development of the circumference of the fibre optics ring, positioned inside the concentrator (9/Tab."A"). This particular layout permits, by the adjustment of the iris diaphragm (3/Tab."A"), to shutter exactly the light beam on an arc portion of the circumference of the fibre optics ring (9/Tab."A"), being thus able to create some shadows on the examined structure. This image, captured by a digital system, permits to measure the shadow and, using some algorithms, to evaluate the exact depth of the structure that has generated it. The whole module can be rotated around its axis to obtain the exact positioning of the lightning arc on the surface to be examined. A second diaphragm with a star-shaped aperture (4/Tab."A") spreads the light evenly on the whole circumference, modifying only the intensity of the light beam. The cylindrical connector, by a specific mechanical adaptor, is connected to different light sources already manufactured and used. The glass support means (8/Tab."A") has a micrometrical adjustment for the exact adjustment of the focusing, can be disassembled for an easy replacement of the glass, and for the cleaning and sterilization of every component in contact with the patient's body. During manufacturing, all the necessary precautionary measures will be applied to make the MODULE comply with existing norms and regulations. The MODULE can be built easily with the building materials and techniques used at present. The MODULE comes into different shapes and dimensions so that it can be used on particular contact areas, characterized by different dimensions and accessibility. This MODULE can be used, with special mechanical adaptors, on every binocular optical system used at present and implemented on those which will be manufactured in the future.

The building philosophy is characterized by the possibility of extending the systems used at present, which, due to their design characteristics, nearly always lack obsolescence, in a way to limit their replacement and to contribute to reduce the production of hazardous waste that require special waste disposal. This philosophical orientation is certainly in contrast with economic interests, but in absolute harmony with environmental protection.

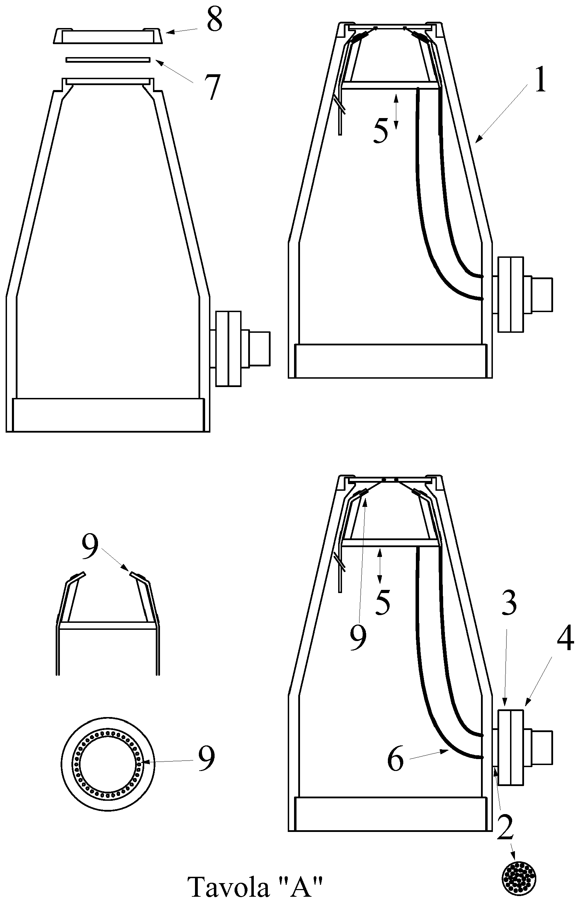

## Claims

1. An illumination module (1) comprising optical fibres (6) in a cylindrical connector (2) and a concentrator (9) in which said optical fibres (6) are arranged in a ring of optical fibres,
**characterized in that**
an iris diaphragm (3) is coupled to said cylindrical connector (2) and
said optical fibres (6) in said cylindrical connector (2) are arranged in a spiral.

2. The illumination module (1) of claim 1 wherein an arc of the ring of optical fibres of the concentrator (9) is illuminated by regulating the iris diaphragm (3).

3. The illumination module (1) of claims 1 or 2 wherein the iris diaphragm (3) has the shape of a ring with a variable area.

4. The illumination module (1) of claims 1 to 3 comprising a further diaphragm having a star shaped aperture (4).

5. An optical system comprising the illumination module (1) of any of claims 1 to 4 and further comprising a stage (8).

6. The optical system of claim 5, wherein the optical system is a microscope.

7. The optical system of claim 5, wherein the optical system is a stereomicroscope.

8. The optical system of any of claims 5 to 7 wherein at least one of the stage (8) and the concentrator (9) are micro-metrically adjustable.

## Patentansprüche

1. Ein Beleuchtungsmodul (1), der enthält optische Fasern (6) in einem zylindrischen Verbinder (2) und einen Konzentrator (9), in dem die erwähnte optische Fasern (6) in einen Ring von optische Fasern aufgestellt sind,
**dadurch gekennzeichnet** bei
einem Irisblende (3), das mit dem erwähnte zylindrischen Verbinder (2) verbunden ist, und, die erwähnte optische Fasern (6) wie eine Spirale in diesen zylindrischen Verbinder (2) aufgestellt sind.

2. Das Beleuchtungsmodul (1) nach Anspruch 1, wobei einen Bogen der Ring von optischen Fasern von Konzentrator (9) beleuchtet ist, durch die Regelung der Irisblende (3).

3. Das Beleuchtungsmodul (1) nach Anspruch 1 oder 2, bei dem die Irisblende (3) die Form eines Ring hat, mit einer veränderlichen Fläche.

4. Das Beleuchtungsmodul (1) nach Ansprüche 1 bis 3, das ein weiteres Irisblende mit Sternförmiger Öffnung (4) enthält.

5. Ein optisches System, die enthält das Beleuchtungsmodul (1) aus nach einem der Ansprüche 1 bis 4, die ein Stütze (8) dazu enthält.

6. Das optische System aus Anspruch 5, in dem das optische System ein Mikroskop ist.

7. Das optische System aus Anspruch 5, in dem das optische System ein Stereomikroskope ist.

8. Das optische System aus einem der Ansprüche 5 bis 7, in dem wenigstens ein aus Stütze (8) und ein aus Konzentrator (9) mit Mikrometrische Einstellung verstellbar sind.

## Revendications

1. Un module d'illumination (1) comprenant des fibres optiques (6) dans un connecteur cylindrique (2) et un concentrateur (9) dans lequel lesdites fibres optiques (6) sont ordonnées en une bague des fibres optiques,
**caractérisé en ce que**
un diaphragme à iris (3) est accouplé avec ledit connecteur cylindrique (2), et lesdites fibres optiques (6), dans ce connecteur cylindrique (2), sont ordonnées dedans une spirale.

2. Le module d'illumination (1) selon la revendication 1, dans lequel un arc de la bague des fibres optiques du concentrateur (9) est illuminé par régulation du diaphragme à iris (3).

3. Le module d'illumination (1) selon la revendication 1 ou la revendication 2, dans lequel le diaphragme à iris (3) a la forme d'une bague avec une superficie variable.

4. Le module d'illumination (1) selon les revendications 1 à 3, comprenant un diaphragme supplémentaire ayant une ouverture en forme d'étoile (4).

5. Un système optique comprenant le module d'illumination (1) d'une quelconque des revendications 1 à 4 et comprenant aussi un support (8).

6. Le système optique selon la revendication 5, dans lequel le système optique est un microscope.

7. Le système optique selon la revendication 5, dans lequel le système otpique est un stereomicroscope.

8. Le système optique selon une quelconque des revendications 5 à 7, dans lequel au moins l'un des support (8) et des concentrateur (9) ont un réglage micrométrique.
